# EUROPEAN PATENT APPLICATION

(11) **EP 4 134 022 A1**
(43) Date of publication of application: **15.02.2023**
(21) Application number: 22169681.8
(22) Date of filing: 11.04.2022
(51) Int. Cl.: A61B 17/22

(54) **LITHOPLASTY BALLOON SYSTEMS, DEVICES AND METHODS WITH ELECTRODE PAIRS HAVING MULTIPLE SPARK GAPS**

(30) Priority: 05.08.2021 US 202163229737 P; 04.10.2021 US 202117449883; 11.11.2021 US 202117454574; 11.11.2021 US 202117454587; 12.11.2021 US 202117454667; 12.11.2021 US 202117454668; 12.11.2021 US 202117454718; 12.11.2021 US 202117454721; 14.12.2021 US 202117644173; 25.03.2022 WO PCT/US2022/071341
(62) Divisional of application: 22167569.7
(71) Applicant: Nextern Innovation, LLC, White Bear Township, MN 55110 (US)
(72) Inventor: BATCHELDER, Sam, White Bear Township, 55110 (US); BALLARD, John R., White Bear Township, 55110 (US); D'AGOSTINO, Robert, White Bear Township, 55110 (US); BRENZEL, Michael P., White Bear Township, 55110 (US); STAAB, Jason W., White Bear Township, 55110 (US)
(74) Representative: Viering, Jentschura & Partner mbB Patent- und Rechtsanwälte

(57) **Abstract**

Various embodiments of the systems, methods and devices are provided for breaking up calcified lesions in an anatomical conduit. More specifically, an electrical arc is generated between two spaced-apart electrodes disposed within a fluid-filled balloon, creating a subsonic pressure wave. In some embodiments, the electrodes comprise a plurality of points that allow the electrical arc to form at any one of the plurality of points to, among other things, extend the electrode life.

## Description

### CROSS-REFERENCE TO RELATED APPLICATIONS

This application claims the benefit of U.S. Provisional Patent Application Serial No. 63/229737, filed August 5, 2021, entitled SYSTEMS, DEVICES AND METHODS FOR GENERATING SUBSONIC PRESSURE WAVES IN INTRAVASCULAR LITHOTRIPSY, U.S. Utility Patent Application Serial No. 17/449883, filed October 4, 2021, entitled SYSTEMS, DEVICES AND METHODS FOR GENERATING SUBSONIC PRESSURE WAVES IN INTRAVASCULAR LITHOTRIPSY, U.S. Utility Patent Application Serial No. 17/454574, filed November 11, 2021, entitled SYSTEMS, DEVICES AND METHODS FOR GENERATING SUBSONIC PRESSURE WAVES IN INTRAVASCULAR LITHOTRIPSY, U.S. Utility Patent Application Serial No. 17/454587, filed November 11, 2021, entitled SYSTEMS, DEVICES AND METHODS FOR GENERATING SUBSONIC PRESSURE WAVES IN INTRAVASCULAR LITHOTRIPSY, U.S. Utility Patent Application Serial No. 17/454667, filed November 12, 2021, entitled METHODS, SYSTEMS AND DEVICES FOR GENERATING SUBSONIC PRESSURE WAVES IN INTRAVASCULAR LITHOTRIPSY, U.S. Utility Patent Application Serial No. 17/454668, filed November 12, 2021, entitled METHODS FOR GENERATING SUBSONIC PRESSURE WAVES IN INTRAVASCULAR LITHOTRIPSY WITH MORE THAN SPARK GAP, U.S. Utility Patent Application Serial No. 17/454718, filed November 12, 2021, entitled SYSTEMS, DEVICES AND METHODS FOR SELECTION OF ARC LOCATION WITHIN A LITHOPLASTY BALLOON SPARK GAP, U.S. Utility Patent Application Serial No. 17/454721, filed November 12, 2021, entitled SYSTEMS, DEVICES AND METHODS FOR MONITORING VOLTAGE AND CURRENT AND CONTROLLING VOLTAGE OF INTRAVASCULAR SUBSONIC LITHOTRIPSY SYSTEMS, and U.S. Utility Patent Application Serial No. 17/644173, filed December 14, 2021, entitled LITHOPLASTY BALLOON SYSTEMS, DEVICES AND METHODS WITH ELECTRODE PAIRS HAVING MULTIPLE SPARK GAPS, the entire contents of which are incorporated herein by reference.

### STATEMENT REGARDING FEDERALLY SPONSORED RESEARCH OR DEVELOPMENT

### BACKGROUND OF THE INVENTION

### FIELD OF THE INVENTION

The invention relates to systems, devices and methods for breaking up calcified lesions in an anatomical conduit. More specifically, an electrical arc is generated between two electrodes disposed within a fluid-filled balloon, creating a subsonic pressure wave.

### DESCRIPTION OF THE RELATED ART

A variety of techniques and instruments have been developed for use in the removal or repair of tissue in arteries and similar body passageways, including removal and/or cracking of calcified lesions within the passageway and/or formed within the wall defining the passageway. A frequent objective of such techniques and instruments is the removal of atherosclerotic plaque in a patient's arteries. Atherosclerosis is characterized by the buildup of fatty deposits (atheromas) in the intimal layer (i.e., under the endothelium) of a patient's blood vessels. Very often over time what initially is deposited as relatively soft, cholesterol-rich atheromatous material hardens into a calcified atherosclerotic plaque, often within the vessel wall. Such atheromas restrict the flow of blood, cause the vessel to be less compliant than normal, and therefore often are referred to as stenotic lesions or stenoses, the blocking material being referred to as stenotic material. If left untreated, such stenoses can cause angina, hypertension, myocardial infarction, strokes and the like.

Angioplasty, or balloon angioplasty, is an endovascular procedure to treat by widening narrowed or obstructed arteries or veins, typically to treat arterial atherosclerosis. A collapsed balloon is typically passed through a pre-positioned catheter and over a guide wire into the narrowed occlusion and then inflated to a fixed size. The balloon forces expansion of the occlusion within the vessel and the surrounding muscular wall until the occlusion yields from the radial force applied by the expanding balloon, opening up the blood vessel with a lumen inner diameter that is similar to the native vessel in the occlusion area and, thereby, improving blood flow.

The angioplasty procedure presents some risks and complications, including but not limited to: arterial rupture or other damage to the vessel wall tissue from over-inflation of the balloon catheter, the use of an inappropriately large or stiff balloon, the presence of a calcified target vessel; and/or hematoma or pseudoaneurysm formation at the access site. Generally, the pressures produced by traditional balloon angioplasty systems is in the range of 10-15atm, but pressures may at times be higher. As described above, the primary problem with known angioplasty systems and methods is that the occlusion yields over a relatively short time period at high stress and strain rate, often resulting in damage or dissection of the conduit, e.g., blood vessel, wall tissue.

Shockwave Medical, Inc., markets an alternative to traditional relatively high pressure balloon angioplasty. The Shockwave Medical, Inc., intravascular lithotripsy system generates "shock waves" within a fluid-filled balloon. Shockwave Medical claims that generated "shock waves" travel at supersonic speed through the balloon fluid, through the balloon material to interact with the vessel wall tissue, stenosis and/or calcification. The Shockwave Medical, Inc., system requires a relatively close spacing between electrodes in an electrode pair wherein the spark gap is disposed. Shockwave Medical's currently known systems provides relatively small axial coverage of lesions. The structure of Shockwave Medical's electrode pairs thus requires additional electrode pairs spaced apart axially from each other and/or a translatable, slidable electrode pair carrier that may be used to translate the electrode pair(s) to better cover an elongated lesion.

Various embodiments of the present invention address these issues, among others, discussed above.

### BRIEF DESCRIPTION OF THE SEVERAL VIEWS OF THE DRAWINGS

These drawings are exemplary illustrations of certain embodiments and, as such, are not intended to limit the disclosure.
FIGURE 1 illustrates a perspective and partial cutaway view of a distal region of one embodiment of the present invention.
FIGURE 2 illustrates a perspective view of a distal region of one embodiment of the present invention.
FIGURE 3 illustrates a perspective view of a distal region of one embodiment of the present invention.
FIGURE 4 illustrates a side, cutaway view of a distal region of one embodiment of the present invention.
FIGURE 5 illustrates a perspective, cutaway view of a portion of a distal region of one embodiment of the present invention.
FIGURE 6 illustrates a side cutaway view of a portion of a distal region of one embodiment of the present invention.
FIGURE 7 illustrates a perspective, cutaway view of a portion of a distal region of one embodiment of the present invention.
FIGURE 8 illustrates a perspective, cutaway view of a portion of a distal region of one embodiment of the present invention.
FIGURE 9 illustrates perspective views of ring electrodes of one embodiment of the present invention.
FIGURE 10 illustrates a side cutaway view of two intermediary electrodes with an operative electrical communication therebetween.
FIGURE 11 illustrates a side cutaway view of two intermediary electrodes with an operative electrical communication therebetween.
FIGURE 12 illustrates a side cutaway view of two intermediary electrodes with an operative electrical communication therebetween.
FIGURE 13 illustrates a side view of simultaneous arcs and a side view with one arc delayed relative to the other arc.

### DETAILED DESCRIPTION OF THE INVENTION

Generally, embodiments of the present invention comprises methods and devices for generating subsonic waves for disrupting or cracking calcified regions within a blood vessel, though the disruptive effects of the generated subsonic waves may extend to partially or non-calcified occluding material. More specifically, with reference to the Figures, an exemplary embodiment 100 comprises an elongated member or carrier 102 such as a catheter with a known inflatable angioplasty balloon 104 mounted on or near the distal end 103 of the elongated carrier 102 which in certain embodiments may comprise a laser cut polyimide tube. The distal end 105 of the balloon 104 may be sealed against or around the elongated carrier 102 to create a watertight barrier and further comprises a fluid inflating/deflating channel 106 in fluid communication with the interior of the balloon 104 and in fluid communication with a fluid-containing reservoir (not shown) that is located external to the patient, and as is well-known in the art, for inflating the balloon 104 with fluid F and deflating balloon 104. A guide wire lumen (not shown but as is well-known in the art) configured to allow translation of a guide wire extends through the elongated carrier and distally out therefrom, an arrangement also well known to the skilled artisan.

It is to be understood that the various embodiments of the present invention are also effective within a fluid-filled environment, e.g., a bodily cavity and/or a blood vessel, i.e., without requiring a fluid-filled balloon. The various embodiments are described in relation to a fluid-filled balloon, but will also apply to an elongated catheter disposed within a fluid-filled environment wherein the subsonic pressure wave generators described infra may be disposed along the elongated carrier within the fluid-filled environment. All such embodiments are within the scope of the present invention.

Therefore, t least one subsonic pressure wave generator 200 is provided, wherein each subsonic pressure wave generator comprises a proximal ring electrode and a distal ring electrode, with a spark gap defined therebetween. In some embodiments, two subsonic pressure wave generators 200, 200' may be provided. In still other embodiments, more than one subsonic pressure wave generator, i.e., two or more, may be provided.

As referred to herein, subsonic pressure wave generator is defined as a mechanism that, when actuated, generates a wave(s) of energy within a fluid-filled environment such as an angioplasty balloon. The generated wave(s) thus travel through the balloon material at subsonic speed and also interact with tissue and/or calcified material located outside of the balloon at subsonic speed. In other words, the wave(s) generated by the subsonic pressure wave generators do not travel through the balloon material or impact tissue or calcified material outside of the balloon at the speed of sound or greater. Further, the term "wave" is not intended to be limiting to a "wave" per se. Instead, a traveling front of energy is generated and that moves through the fluid within the balloon, generally away from the subsonic pressure wave generator from which it eminates. This traveling front of energy may comprise a symmetrical expansion shape around the elongated catheter 102, or may expand and travel in an asymmetric shape relative to the elongated catheter 102. In each embodiment, the traveling front of energy, i.e., the "wave" as referred to herein, travels through the balloon material and impacts materials outside of the balloon at subsonic speeds.

Alternatively, the subsonic pressure wave generator may comprise a resistive heater or a pulse heater as is known in the art.

If a single subsonic pressure wave generator 200 is provided, it may be substantially axially centered within the balloon 104. In other embodiments, the single subsonic pressure wave generator 200 may be biased to the proximal or to the distal end of the balloon's interior.

When two or more subsonic pressure wave generators are provided, 200, 200', adjacent subsonic pressure wave generators, e.g., 200, 200', may be spaced axially apart from each other, wherein the resultant spark gaps defined by each subsonic pressure wave generator 200, 200', are axially spaced apart from each other. In cases wherein three or more subsonic pressure wave generators are provided, the resultant spark gap between adjacent subsonic pressure wave generators may be substantially equal, or one or more spark gaps may be longer or shorter than other subsonic pressure wave generators.

As further seen in the Figures, a first, proximal, subsonic pressure wave generator 200 may comprise a proximal ring electrode 201 and an axially spaced apart distal ring electrode 202, defining a spark gap therebetween. Next, a second, more distal, subsonic pressure wave generator 200' may comprise a proximal ring electrode 203 and an axially spaced apart distal ring electrode 204, also defining a spark gap therebetween. As will be discussed further, the distal ring electrode 202 of subsonic pressure wave generator 200 and the proximal ring electrode 203 of subsonic pressure wave generator 200' may be in electrical communication with each other to enable current to flow therebetween.

As will be understood by skilled artisan, the electrical communication may be effectively reversed. First, e.g., with a proximal electrode electrically coupled or in electrical communication with a "high" power side of a circuit and pulse generator connected therein, and a distal electrode electrically coupled or in electrical communication with a "ground" or "return" side of the circuit and pulse generator connected therein. Second, a distal electrode may be electrically coupled or in electrical communication with a "high" power side of a circuit and pulse generator while a proximal electrode may be electrically coupled or in electrical communication with a ground or return side of the circuit and pulse generator. In either case, once the subsonic pressure wave generator(s) is/are actuated, the circuit is completed and current will flow through the circuit.

At least one of the subsonic pressure wave generators, e.g., 200 may be in direct electrical connection and communication with an externally located power source or pulse generator 300, wherein the pulse generator may be configured to provide voltage pulses of a predetermined magnitude and pulse length along an electrical conductor to a proximal ring electrode of a proximal-most subsonic pressure wave generator 200. Alternatively, the voltage pulses may be delivered without a predetermined magnitude or pulse length. In some embodiments, a collapsing field in an inductor, e.g., a well-known car ignition mechanism), or decaying voltage from a capacitor may be employed, neither of which comprise or require a predetermined voltage or pulse length.

Each subsonic pressure wave generator 200, 200', etc., comprises a pair of axially spaced-apart ring electrodes. Electrode pairs 201, 202 and 203, 204 are shown in axially spaced-apart disposition and mounted around the elongated carrier 102, e.g., by crimping or other attachment means and are immersed within the fluid F in the inflated balloon 104. Accordingly, spark gaps are defined between electrode pair 201 and 202, and between electrode pair 203 and 204, wherein electrodes 202 and 203 are in operative electrical communication or connection. As discussed above, the spark gaps may be of equivalent length or may comprise differing lengths. In some embodiments, a single subsonic pressure wave generator 200 may be provided, while in other embodiments, more than one subsonic pressure wave generator 200, 200', etc., may be provided.

Thus, in some embodiments, first and proximal-most ring electrode 201 may be electrically coupled or in electrical communication or connection, via an electrical conductor, with a power source, e.g., the pulse generator 300, that is configured for supplying voltage pulses to the electrode pair(s) comprising the subsonic pressure wave generator(s) 200. The distal-most ring electrode, e.g., 204, may also be electrically coupled or in electrical communication or connection, via a second electrical conductor, with the power source, e.g., pulse generator 300.

The fluid F within the inflated balloon 104 is ionically conductive, e.g., saline, to facilitate arcs, or current flow, between the spaced-apart ring electrodes in each electrode pair 201, 202 and 203, 204 comprising the subsonic pressure wave generators 200 and 200'. Thus, upon application of sufficient voltage generated by the pulse generator 300 to the proximal-most electrode, e.g., 201, via a conductor in electrical connection or communication between pulse generator 300 and electrode 201, may cause current to flow between electrode 201 and electrode 202 and wherein an arc is generated across the defined spark gap between electrodes 201, 202. A return conductor in operative electrical connection or communication with electrode 202 completes the circuit back to the pulse generator 300. In this manner, the circuit may complete or close during the arcing between ring electrodes 201, 202 in an embodiment having a single electrode pair comprising a single subsonic pressure wave generator 200.

It is known that current can flow between the electrodes without an arc. Current generally flows in an electrolyte by ion diffusion. An arc or spark is present when electrons or ions can accelerate past the ionization energies of the local molecule, creating a cascade. Often, this is a plasma and may occur through the bulk fluid, e.g., the conducting fluid F, but is more likely to occur along a fluid-surface interface, e.g., along the outer surface of the catheter 102. These conditions may also result in generation of subsonic pressure waves as described above.

In an embodiment comprising more than one subsonic pressure wave generator 200, 200' such as illustrated, upon application of sufficient voltage by the pulse generator 300, the current flow (arcing) may proceed from electrode 201 to electrode 202 across the defined spark gap therebetween. Next, electrode 202, being in operative electrical communication with electrode 203, enables current to flow from electrode 202 to electrode 203 which, in turn, results in current flow from electrode 203 to electrode 204, across the spark gap defined therebetween. A return conductor in operative communication with electrode 204 completes the circuit back to the pulse generator 300.

The flow discussed above comprises a "current" passing from electrode 201 to 202 is initially ion diffusion as discussed above (before the arc is established), followed by streamers initiating from one or more points 206 of electrode 201, followed by plasma channels being formed either through the fluid F and/or at, or along, the fluid F surface interface. The fluid F surface interface may comprise the outer surface of catheter 102 and/or the inner surface of angioplasty balloon 104.

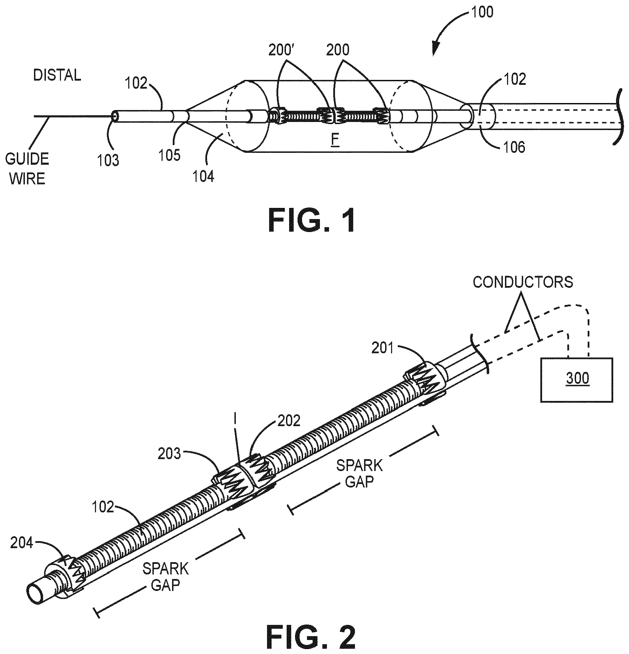

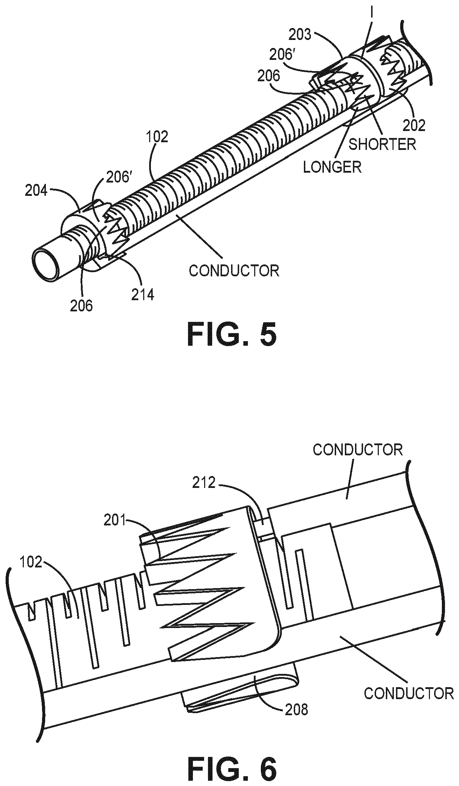

Fig. 1 illustrates the fluid-filled balloon 104 in an inflated state wherein a conductive fluid F such as saline fills the balloon's interior space, with the spaced-apart ring electrodes 201, 202 and 203, 204 disposed therein and immersed in fluid F. Electrodes 201, 202, 203 and 204 are arranged generally symmetrically around the elongated carrier 102 and generally symmetrically along a center line of the inflated balloon 104. However, in a preferred embodiment, as shown in at least Fig. 6, a channel 208 may be defined through or along the ring electrodes along a longitudinal plane to allow the insulated conductor(s) to be disposed at least partially therein so as to reduce crossing profile of the system. Thus, the channel 208 may be formed by carving out a portion of ring electrode wherein the ring electrode does extend circumferentially around the elongated carrier 102. Alternatively, as illustrated in Fig. 6, channel 208 may comprise a void or space between two spaced-apart ends of the ring electrode, wherein the ring electrode extends partially circumferentially around the elongated carrier 102 and wherein the conductor may extend along the outer surface of elongated carrier 102. With the exception of the interruption of the channel 208 in the ring electrode(s), the preferred structure is symmetrical as discussed above, though asymmetrical electrode(s) may also be employed.

Figures 2-12 illustrate possible arrangements and embodiments of the spaced-apart ring electrodes that form each electrode pair as well as the conductive wire connections thereto.

Fig. 2 thus illustrates the elongated carrier 102, which may comprise a laser cut tube and may comprise polyimide or other material. Two exemplary subsonic pressure wave generators 200, 200' are shown in axially spaced-apart relation relative to each other along the elongated carrier 102. Each subsonic pressure wave generator, e.g., 200, 200', comprise spaced-apart exemplary ring electrodes, respectively 201, 202 and 203, 204, each defining a spark gap between the relevant spaced-apart ring electrodes of a predetermined length, that is the spacing distance between the spaced-apart ring electrodes 201 to 202, and 203 to 204. The distal ring electrode, e.g., 202, of the proximal subsonic pressure wave generator 200 and the proximal ring electrode 203 of the distal subsonic pressure wave generator 200' are shown in relatively close disposition forming an interface I therebetween, the interface defining and comprising an electrical communication between the two ring electrodes defining the interface I.

The various forms and types of electrical connections between these intermediary ring electrodes 202, 203 defining an interface I are described further herein, but generally comprise a physical or operative electrical connection between surfaces of the two intermediary ring electrodes that may comprise a touching relationship, a weld bead, or a jumper wire or other conductive interconnection element, or mechanism, between the two intermediary ring electrodes 202, 203, or other conducting connection. The skilled artisan will readily recognize alternative mechanisms for creating the required electrical connection between the intermediary ring electrodes, 202, 203 i.e., between adjacent subsonic pressure wave generators 200, 200', each of which is within the scope of the present invention. In this arrangement, the two or more subsonic pressure wave generators 200, 200', etc., may be electrically connected in what effectively becomes a series circuit. The number of subsonic pressure wave generators used in certain embodiments may be one, or two, or more than two.

As discussed further herein, the ring electrodes described herein are exemplary, other electrodes shapes and structures are within the scope of the present invention. In certain embodiments, and as discussed further *infra*, at least one of the electrodes in an electrode pair comprising a subsonic pressure wave generator may comprise a plurality of points or extensions that extend toward the spark gap defined between the electrode pair.

Still further, certain embodiments may comprise a plurality of electrode pairs, at least one electrode pair comprising a proximal-most ring electrode in wired, or other, electrical communication with the pulse generator 300. In some embodiments, more than one electrode pair in the plurality may comprise a proximal-most ring electrode in wired, or other, electrical communication with the pulse generator 300, wherein at least one of the electrode pairs in the plurality may be separately and individually energized by the pulse generator 300. Thus, certain embodiments may comprise a parallel connection arrangement of at least some electrode pairs, or may comprise a combination of series connected sets of electrode pairs with one or more sets of electrode pairs comprising a parallel connection back to pulse generator.

The skilled artisan will recognize that the reference to an operative electrical connection or communication with a proximal-most ring electrode of an electrode pair and the pulse generator 300 is merely illustrative. It is within the scope of the present invention to simply switch the operative electrical connection to be between a distal-most ring electrode of an electrode pair and the pulse generator 300.

In certain configurations, individual subsonic pressure wave generators, 200, 200' may be controlled regarding the magnitude of voltage applied, the magnitude of current flow resulting in an arc between the ring electrodes comprising the subsonic pressure wave generators, the time duration of current flow and arcing between the ring electrodes comprising the subsonic pressure wave generators, the current in the primary of a discharge inductor, the charge in a discharge capacitor and/or the initiation time of the current flow or arcing between the ring electrodes comprising the subsonic pressure wave generators.

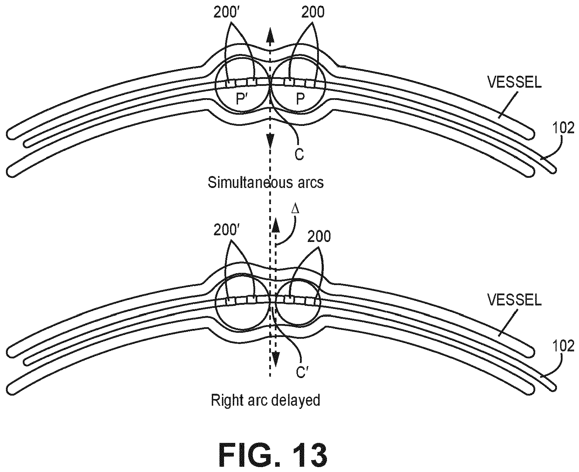

For example, and with reference now to FIG. 13 and application of the related detailed description *infra*, is possible to axially translate or shift a central node between generated pressure waves by slightly delaying generation of one pressure wave by one or more adjacent subsonic pressure wave generators, e.g., 200 or 200', relative to the timing of generation of a pressure wave by an adjacent subsonic pressure wave generator, such variable gap spacing may also provide an alternative, or supplemental, mechanism for moving the resulting pressure waves, and nodes disposed therebetween, axially along the catheter 102 within balloon 104. The delay in pressure wave generation may be used alone, or in combination with the axial spacing differentials between adjacent subsonic pressure wave generators 200, 200'.

As shown in Figure 13, two (or more) pairs of ring electrodes, 201/202 and 203/204 may be provided within the fluid-filled balloon. The arcing for each pair 201/202 and 203/204 may be generated substantially simultaneously, resulting in equal-sized bubbles at any given time and subsonic pressure waves P with a central node C generally in the middle of the generated subsonic pressure waves P.

Alternatively, one arc (and resultant subsonic pressure wave P) may be slightly delayed which is used to shift the central node C proximally or distally to enable treating along the axial length of the balloon. Figure 13 illustrates the axial offset Δ of the central node C vs C' as a result of this delay technique. Such a delay in arcing, and resulting subsonic pressure wave P' which is slightly delayed relative to subsonic pressure wave P, may be timed and used to create a sweeping effect of a axially translating pressure wave through the length of the balloon and along the length of the lesion. A processor may be provided as well-known in the art to execute a pre-programmed set of instructions comprising various timing sequences of the pulses and resulting arcs and pressure waves to optimize focus of the waves including, but not limited to sweeping the lesion in axial directions. As shown, the pairs of interacting ring electrodes 201/202 and 203/204 are adjacent each other along the elongated carrier. In other embodiments, non-adjacent ring electrode pairs may interact as discussed above.

### Catheter and Electrodes

As provided above, an exemplary laser-etched polyimide tube 102 may be provided with ring electrodes 201, 202 and 203, 204, wherein the ring electrodes are crimped around the tube, with insulated wires connecting the ring electrodes back to the external pulse generator 300.

In the two-wire configuration shown, the gap between the electrodes may be decreased by opening the distance between the two adjacent center, intermediary electrodes (202 and 203) in the electrode pairs while electrically connecting them with an additional wire.

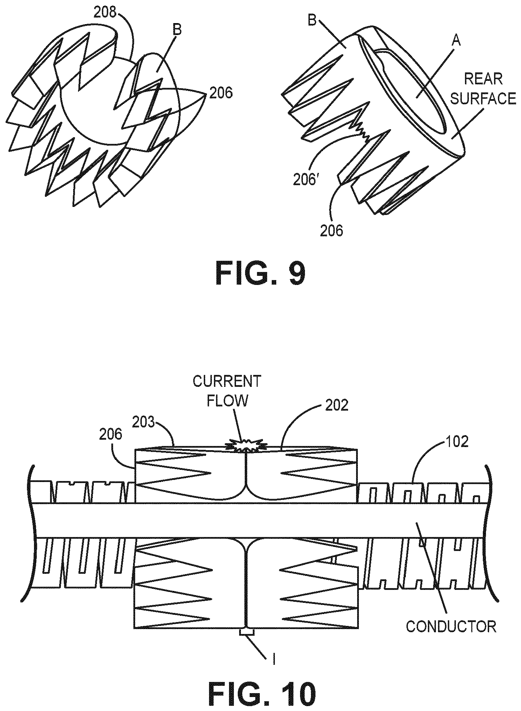

Figure 9 provides an exemplary ring electrode E having a body portion B defining a central aperture A configured to securely engage the catheter 102, channel 208, a front surface defining a plurality of points 206 and a flat rear surface. Points 206' illustrate exemplary effects of corrosion on one of the points caused by arcing between adjacent ring electrodes. One or more of the remaining points 206 may engage to generate the arc across the spark gap.

The points 206 may comprise a substantially triangular profile as illustrated. However, other profiles are also contemplated. The underlying functionality of the points 206 is to enable arcs to initiate from different locations on the electrode. Therefore, any shape that extends away from the main body of the electrode generally toward the distal-most electrode in an electrode pair, and generally toward the spark gap defined therebetween, comprising a subsonic pressure wave generator will be sufficient. The tip regions of adjacent ones of the plurality of points are in certain embodiments, spaced apart from each other.

Multiple points 206 on the exemplary ring electrodes facing the spark gap region defined between ring electrodes, e.g., 201, 202, allow electrical breakdown streamers to initiate from several different locations or points 206 disposed on and/or around the ring electrode, so viable points 206 remain when some are corroded by the arc. This extends the effectiveness and life of the ring electrode. In addition, the path of the arc may comprise debris, so originating arcs from different locations, i.e., points 206, on the electrode(s) aids in reducing the debris, making it less likely that a short is formed. In this way, the environment surrounding the electrodes and within the spark gap therebetween is maintained as uniformly as possible throughout the treatment session comprising a plurality of pulses.

Accordingly, as illustrated in the Figures, and as the skilled artisan will readily understand, the uncorroded point(s) 206 involved in electrical arcs, begin to corrode as electrical arcing proceeds. As shown in Figs. 5 and 9, points 206 corrode to shorten to form degraded or corroded points 206'. In turn, as will be understood and illustrated, the spark gap between corroding, or corroded, points 206' will lengthen, creating a greater length of fluid and distance, and resistance, therebetween. Thus, the current flow streamers may continually seek out a shorter, less resistant, spark gap formed or defined by, or between one or more uncorroded points 206 that are longer in length that corroded point(s) 206'. Relatedly, in some embodiments, as best shown in Fig. 5, one or more of the uncorroded points 206 may have a length that is longer than one or more of the other points 206, as measured by the point(s) 206 relative length of extension toward the spark gap. The longer point(s) 206 thus comprise a spark gap length that is shorter, and less resistant, than the spark gap length of other point(s) 206 that are shorter, or the spark gap length of points 206' that are corroded and, therefore, shortened to define a longer spark gap length therebetween. Fig. 5 shows an exemplary set of points 206 wherein one point 206 is "longer" than an adjacent "shorter" point 206 and a still shorter point 206' that has been shortened by corrosion by electrical arcing. As the skilled artisan will readily understand, current flow streamers may preferably seek out a shorter, less resistant, spark gap, i.e., a spark gap comprising one or more "longer" points 206.

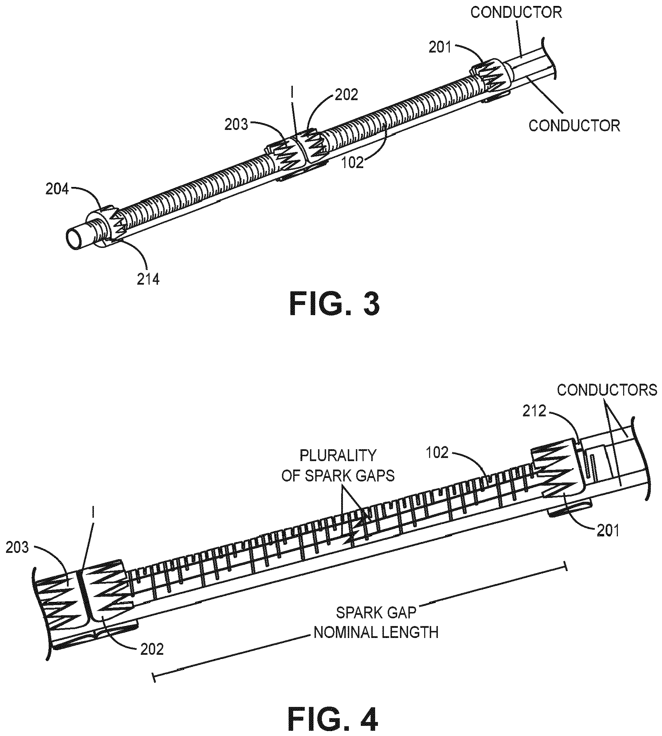

As shown in Fig. 3, the extensions or point(s) 206 of the electrode pairs, e.g., 201, 202 may be configured to define a plurality of spark gaps therebetween, each spark gap in the plurality corresponding with a pair of opposing extensions or points 206 that are longitudinally aligned between the exemplary spaced-apart electrodes 201, 202. In this manner, as one spark gap lengthens due to corrosion as described herein, the current streamer formation may move to another pair of longitudinally aligned opposing extensions or points 206 that are uncorroded and, therefore, in some embodiments, defining a spark gap that is shorter than the spark gap that has lengthened due to corrosion of the relevant opposing extentions or points 206. In addition, and as shown, the extensions or points 206 of the electrode pairs, e.g., 201, 202, are radially spaced apart from each other around the relevant electrode 201 and/or 202. Accordingly, and as further described herein, the corresponding spark gaps therebetween are also radially spaced apart. As a result, a first electrical arc, and the corresponding first pressure wave generated by the first electrical arc, across a first spark gap may occur at a first radial location around the electrodes 201, 202 and around elongated member or carrier 102 . A subsequent electrical arc, and its generated pressure wave, may occur at a second radial location around exemplary electrodes 201, 202 and that is spaced apart from the first radial location.

The electrodes, including exemplary ring electrodes 201, 202, 203, 204, may be metal or semiconductor, and can be plated with a secondary alloy. The base metal may comprise copper or beryllium copper. The plating may comprise platinum, gold, tungsten, osmium, silver, nickel, or other electrochemically low-activity metal. Carbon surfaces such as graphite, graphene, and diamond may also be used. Still further, stainless steel and steel alloys may be used.

The connection between electrode pairs, e.g., 201, 202 and 203, 204, may be achieved in many embodiments. As discussed above and as shown in Figure 10, in one embodiment, the two intermediary ring electrodes, e.g., 202 and 203, may be placed in a physically touching relationship wherein the electrical connection effectively comprises a short between the touching electrodes 202, 203, allowing current to flow therebetween. The electrode rings 201, 202, 203, 204 may comprise a rear surface (shown in Fig. 9) that may be substantially flattened, wherein the rear surfaces of intermediary ring electrodes 202, 203 may be in a physically touching engagement. Alternatively, the rear surfaces of exemplary intermediary ring electrodes 202, 203 may be spaced apart as further discussed here. Still more alternatively, the rear surfaces of the intermediary ring electrodes may comprise complementary shapes, e.g., one convex and the other concave, wherein one rear surface fits within the other rear surface to comprise a fuller physically touching engagement between the intermediary ring electrodes, e.g., 202, 203. The rear surface which may be relatively flattened comprises the side opposite the plurality of points 206 which form and define a front surface of each exemplary ring electrode 201, 202, 203 and 204.

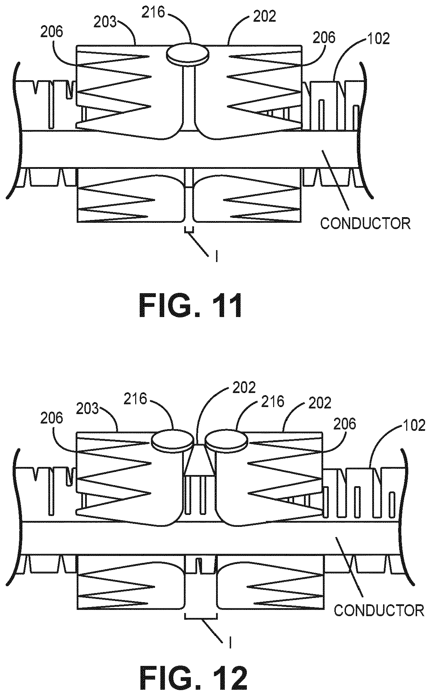

As shown in Figure 12, rear surfaces of intermediary electrodes 202, 203 may be configured in an adjacent but spaced, apart and non-touching engagement, wherein a jumper conductive wire is disposed between the intermediary electrodes 202, 203 across interface I, or, as in Figure 11, a welded bead interconnects the electrodes 202, 203 at the interface I. Alternative means to achieve the required electrical connection at the interface I between intermediary ring electrodes 202, 203 may appear to the skilled artisan, each such electrical connection means is within the scope of the present invention.

Alternative electrode embodiments comprise at least some non-ring electrodes attached or mounted or connected with the elongated catheter 102, wherein pairs of the non-ring electrodes are arranged in spaced-apart configurations to form subsonic pressure wave generators as described above in connection with the ring electrode embodiments. Ring and non-ring electrodes may be combined in a given system.

Still more alternatively, at least some of the electrodes may be disposed along the inner surface of the balloon 104. In certain embodiments a proximal electrode, e.g., a ring electrode such as 201 may be provided and mounted on or along catheter 102, and paired with an electrode disposed along the inner surface of balloon 104. As voltage pulses are applied, an arc may generate between the catheter-mounted electrode and the balloon-mounted electrode, generating in turn subsonic pressure wave(s). Still further, a distal catheter-mounted electrode, e.g., ring electrode 202, may be spaced away from both the proximal catheter-mounted electrode and from the balloon-mounted electrode. In this embodiment, a first subsonic pressure wave may result from an arc between the proximal catheter-mounted electrode and the balloon-mounted electrode. A second subsonic pressure wave may then result from an arc between the balloon-mounted electrode and the distal catheter-mounted electrode. A heat shield may be disposed along and/or around the region where the balloon-mounted electrode is positioned to aid in heat dissipation and conduction of generated heat away from the balloon material.

Finally, the subsonic pressure wave generators may all be mounted along the inner surface of the balloon, with arcs and resulting subsonic pressure wave generation as described herein.

Electrodes mounted on the inner balloon surface may comprise a carbon filament in operative communication with a pulse generator and which may also affect, e.g., limit, the expansion radius of the balloon.

In all of the cases, a plurality of points 206 may be provided on at least one of the electrodes in an electrode pair comprising a subsonic pressure wave generator.

The plurality of points 206 will also help in cases where the elongated catheter 102 is in a curved disposition due to the tortuosity of the subject vessel. In this situation, the points 206 of the subject electrodes in an electrode pair that are on an inner radius of the curved catheter 102 are in closer proximity to each other than the points 206 on an outer or intermediary radius. Thus, these points 206 that are in closer/closest proximity will be likely to generate the arc and resultant subsonic pressure wave.

Further, it is possible to create a preformed curvature in the catheter 102 in order to effectively select which points 206 are likely to generate the arc and resulting subsonic pressure wave. Such a preformed curvature may be built into the catheter 102 using a mandrel and heat setting or other known techniques and/or shaping metal alloys such as Nitinol. One of more of these preformed curvature region(s) may be located along the section inside the balloon 104. This deformation or curvature may be straightened by translation over the guide wire, and subsequent withdrawal of the guide wire allows the subject preformed curvature region to successively move from a deformed straightened configuration to a non-deformed and curved configuration. As will now be apparent, more than one of these preformed curved regions may be provided within the balloon and may be positioned adjacent to electrodes, within or along electrode pairs and/or subsonic pressure wave generators. The preformed curved regions may take curved excursion paths that are in a same direction, or in different directions and may be interposed with straight non-curved sections. In this way, the operator may effectively change the direction of the pressure wave to create more effective disruption of the targeted region.

In certain embodiments, individual points 206 may be specifically energized with individual wired connection(s) and/or individual points 206 may be de-energized in order to ensure they do not participate in current flow, for at least a period of time and/or during treatment of a certain region of the subject vessel.

In other embodiments, the points 206 may be selectively and intentionally degraded (or not degraded) based on material selection and/or relative length of the tip of certain of the points 206 relative to the other points 206.

### Wiring/Cabling

The disposable catheter assembly may comprise two or more insulated conductors connecting the system of electrodes, electrode pair(s) and/or subsonic pressure wave generator(s) to the power supply. A typical excitation pulse is 50A @ 2KV for 5usec, requiring a load impedance of 40 ohms. The round trip cable length in the disposable catheter is approximately 10 feet, so the maximum resistance of the cable is 2 ohms/foot for each trace.

Twisted wire pairs may form transmission lines whose characteristics change with the wire diameter and spacing. If, for example, 40ga copper wire is spaced 0.25mm (10mils) apart (for 5mil thick insulation), the twisted wire pair may form a 1.1uH inductor which may, in turn, cause the rise time of an ideal 50A 2KV source to be about 25nsec. Alternatively, larger, more conductive wire may be used and a resistance may be added to the circuit to accommodate the ideal resistance in the system.

The Figures illustrate electrical conductors comprising insulation that are operatively connected with the pulse generator 300 and wherein one of the electrical conductors is in electrical communication with the proximal-most ring electrode 201, an electrical structure well-known to the artisan. Figure 4 provides an exemplary connection embodiment wherein a distal end of conductor is stripped of insulation exposing a length of distal conductor portion 212 that is operatively connected with ring electrode 201. A similar connection mechanism may be employed for the connection between the other electrical conductor and the distal-most ring electrode, e.g., element 204.

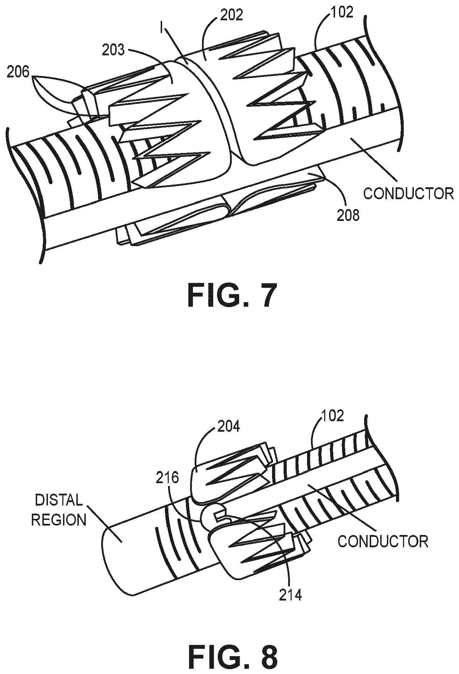

Alternatively, a conductor may comprise a distal conductor portion 214 that is stripped of insulation and that is connected with the relevant ring electrode by a weld bead 216 as shown in Fig. 8. Any of the electrical conductors may be connected to the relevant ring electrode in this manner.

In order to minimize outer diameter and crossing profile of the system, the electrical conductors may be run within a lumen defined in catheter 102, wherein the distal conductor portion is operatively connected with the relevant ring electrode through an aperture in the catheter 102 and/or via a weld bead as described above.

Alternatively and as shown in the Figures, the ring electrodes 201, 202, 203, 204, may comprise a channel 208 sized for the electrical conductor(s) to reside within. The channel 208 may provide the connection point for one or more of the ring electrodes as is shown in, e.g., Figure 8. Channel 208 may allow the electrical conductor(s) to slide there along to accommodate changes in the attitude of the catheter 102 during advancement of the device 100 through a patient's vasculature.

Still more alternatively, a longitudinal channel or a spiral or other shaped channel may be defined in the wall of elongated catheter 102. The conductor(s) may be at least partially disposed in the channel to assist in minimizing crossing profile of the system.

### Power Supply / Pulse Generator

In some embodiments, a capacitor bank may be provided and may be charged during an exemplary 1-minute off period, followed by a short or connection of the capacitors to the electrodes for the discharge and arc generation. The charging period may be less than 1-minute in preferred embodiments. In other embodiments, a current may be established in a transformer primary, wherein that current is halted to generate a large voltage across the secondary.

As noted, the charging period may be much less than 1 minute as a pulse may be delivered to the electrodes at least once a second. The pulse rate may be limited with sensed temperature of the conductive fluid F and/or balloon material so that the temperature of surrounding tissue is not increased beyond a predetermined threshold, e.g., 1 degree C of temperature increase for cardiac tissue. The temperature may be monitored using a temperature sensor mounted along the outside surface of the catheter 102 within the conductive fluid F and/or on an inner surface of the balloon, or other location. The temperature sensor may be in operative communication with an externally located processor having operational communication with the predetermined heat threshold(s) and wherein an alert is provided via a display or other mean. In some embodiments, the voltage pulses may be locked out, with no further pulses allowed. In other embodiments, no further voltage pulses are allowed when the predetermined heat threshold is met or exceeded, but the voltage pulses may proceed when the sensed temperature drops below the predetermined heat threshold.

The capacitor bank may be charged from either direction and FETs are controlled to allow the capacitor banks to discharge between the electrodes in an H-bridge configuration. In some embodiments, the current sign may be configured to flip. Phase shaping may be executed to reduce EMI in some embodiments. In some embodiments, both the current and voltage may be monitored to inform what the voltage setting should be for the next pulse delivery. In some embodiments, the voltage may be terminated on a pulse-by-pulse basis and in other embodiments the voltage is not terminated on a pulse-by-pulse basis. Similarly, the electrical arc across a given set of electrodes comprising a subsonic pressure wave generator may be terminated on a pulse-by-pulse basis in some embodiments, while in other embodiments, said electrical arc may not be terminated on a pulse-by-pulse basis.

Because the treatment scales with the cube root of the deposited energy, casual control of voltage and current suffices. The current may flip sign between pulses, droop or exponentially decay during the pulse, and ring or oscillate during the pulse. It is most efficient that the electrical energy be delivered to the electrodes comprising the subsonic pressure wave generator(s) while the balloon fluid F comprises a mass density that is relatively high, roughly in the first 1-20usec.

The current and voltage output may be monitored for proper operation. Measuring opens or shorts may produce a prompt or alert to change a catheter assembly for a new catheter assembly. Monitoring the DC impedance between the electrodes, e.g., 201 and 202, and the patient allows catheter insulation leaks to be sensed and corrected. As further described herein, monitoring the DC resistance between the electrodes may provide a temperature monitor. Still further, if the vessel is successfully being opened by treatment, the DC resistance between the electrodes decreases because of the larger cross section of saline conducting between the electrodes. It is further understood that as gas is produced from the arcs, the resistance will change.

Further, sensing and/or monitoring the conductivity of the conducting fluid F within the balloon alone, or comparing same with the conductivity of fluid, e.g., blood, outside of the balloon provides alternative mechanisms for determining whether the balloon has been compromised, e.g.,. a rupture or tear.

The patient's heart rhythm may be monitored, and that these pulses are synchronized to an inactive phase. That synchronization precludes some standard methods, such as a spark gap that closes when the capacitor bank reaches a target voltage. Relatedly, the balloon 104 will expand and contract with a characteristic time and frequency. Voltage pulses may be timed to take advantage of the natural expansion/contraction cycle and frequency. For example, voltage pulses may be timed to the natural expansion of the balloon and/or to the natural contraction of the balloon. The force of the subsonic pressure waves will impact the target tissue and/or occluding material, e.g., calcification, at slightly different angles depending on the balloon's expansion state, because, *inter alia*, the subsonic pressure wave generators position will change with expansion/contraction of the balloon.

### Temperature Sensor

As discussed above, certain embodiments may comprise a small temperature sensor embedded near the electrodes and/or within the conductive fluid F which may increase the treatment pulse rate up to the limit of a safe rise in tissue temperature - generally local tissue temperature should not be increased more than about 1 degree C. Heat diffusion on the order of 5mm from the electrodes is required for the heat to be convected by blood circulation. The thermal diffusion time for water at in conduits of relevant radius range is (5mm)2/k = 167 seconds. However, a 0.5J pulse raises a 5mm radius sphere of water approximately 0.23 degrees C, so a 1-pulse/spark-per-minute rate may be increased to 2-pulses/sparks-per minute in certain embodiments.

The temperature sensor may be optical fiber based, or a micro-thermocouple. Since saline increases conductivity with temperature, the current produced by a DC bias applied to the electrodes will increase monotonically with temperature, allowing the temperature of the warmest region to be measured directly. As described above, a predetermined heat or temperature increase threshold may be provided with subsequent alerts and/or corrective or remedial actions implemented by programmed instructions implemented by a processor.

### Balloon and Inflation Liquid

Angioplasty balloons are developed and nuanced. Embodiments of the present invention comprise standard angioplasty balloons and related, and known, basic inflation/deflation mechanisms. A typical balloon length may be 12mm and may be used with 0.14-0.35in guide wires. The inflated balloon size may comprise about 90% of the nominal vessel size.

Varying the salinity of the water used to inflate the balloon has an impact on the breakdown voltage between the electrodes similar to their spacing. Thus, electrode spacing to form a subsonic pressure wave generator may be selected to be appropriate for standard saline, or when a lower-than-saline salt concentration used to inflate the balloon, the electrode spacing may be increased past that used for standard saline. The current density prior to arc formation may be 50A through 0.1cm², or about 500A/cm² at 2,000V, so an initial saline concentration should be at least 2.0E-4M NaCl. Standard saline is 0.9% NaCl, or 1.5E-1M, approximately 1000× more concentrated than required to initiate an arc.

The voltage pulse generated by the pulse generator 300 generates streamers in the fluid F interposed between, e.g., the proximal ring electrode 201 and the next more distal ring electrode 202 that comprise a subsonic pressure wave generator 200. As described above, the distal-most ring electrode is also operatively connected with the pulse generator 300. Sufficient voltage applied to the proximal ring electrode 201 results in streamers and ultimately current flowing between the two ring electrodes of the electrode pair 201, 202, generating an arc and a resultant subsonic pressure wave as a bubble forms and expands in the fluid F, and another subsonic pressure wave as the bubble collapses. Generally, the expansion time for the bubble expansion may be measured in terms of microseconds, e.g., approximately 30 microseconds. This expansion time is slow compared to the transit time of sound across the bubble. "Shock waves" require generation of pressure waves that travel at or greater than the speed of sound.

We note here that this relatively slow expansion time, *inter alia*, ensures that the pressure wave generated is subsonic. In contrast, an actual shock wave, i.e., traveling at or greater than the speed of sound, is generated with a much shorter voltage pulse, on the order of tens of nanoseconds.

The distance between ring electrodes of an electrode pair, e.g., 201, 202 may be relatively long, e.g., 5mm or longer. In this case, the generated bubble and resulting pressure wave may comprise cylindrical shapes, with the end portions of each more spherical in shape.

In the following, various aspects of this disclosure will be illustrated:

Example 1 is a subsonic pressure wave generating angioplasty catheter. The subsonic pressure wave generating angioplasty catheter may include an elongated carrier; an angioplasty balloon comprising a material and disposed near a distal end of the elongated catheter, wherein a distal end of the angioplasty balloon is sealed against the elongated catheter, the angioplasty balloon defining an interior region; a fluid channel in fluid communication with the interior region of the angioplasty balloon and a conductive fluid reservoir, configured to inflate the balloon with the conductive fluid; a first subsonic pressure wave generator disposed along the elongated carrier and within the interior region of the balloon and comprising a first proximal ring electrode and a first distal ring electrode spaced axially from the first proximal ring electrode; and a pulse generator in electrical communication with the first subsonic pressure wave generator, wherein application of a voltage pulse from the pulse generator to the first subsonic pressure wave generator is configured to generate an electrical arc through the conductive fluid and generation of subsonic pressure waves that pass through the balloon material at subsonic speed.

In Example 2, the subject matter of Example 1 can optionally include that the catheter further includes a second subsonic pressure wave generator further comprising a second proximal ring electrode and a second distal ring electrode, the second distal ring electrode spaced axially from the second proximal ring electrode, and disposed along the elongated carrier, within the interior region of the angioplasty balloon, wherein the second subsonic pressure wave generator is in operative electrical communication with the first subsonic pressure wave generator, and wherein the second subsonic pressure wave generator is configured to receive a voltage pulse from the pulse generator and further configured to generate an second electrical arc through the conductive fluid and subsonic pressure waves that pass through the balloon material at subsonic speed.

In Example 3, the subject matter of Example 2 can optionally include that the second subsonic pressure wave generator is axially spaced from the first subsonic pressure wave generator.

In Example 4, the subject matter of Example 2 can optionally include that the first distal ring electrode of the first subsonic pressure wave generator and the second proximal ring electrode of the second subsonic wave generator comprise intermediary electrodes, wherein the intermediary ring electrodes are in operative electrical communication with each other.

In Example 5, the subject matter of Example 4 can optionally include that intermediary ring electrodes are not spaced apart and are physically engaged with each other at an interface.

In Example 6, the subject matter of Example 2 can optionally include that the intermediary ring electrodes each comprise a rear surface, wherein the intermediary ring electrodes are located such that the rear surfaces arranged in a physically touching engagement at an interface.

In Example 7, the subject matter of Example 6 can optionally include that the rear surfaces of the intermediary ring electrodes are substantially flat.

In Example 8, the subject matter of Example 5 can optionally include that the rear surfaces of the intermediary ring electrodes comprise complementary shapes and wherein the complementary shapes of the rear surfaces are arranged to provide a physical engagement at an interface having a surface area that is greater than a physical engagement between rear surfaces that are substantially flat.

In Example 9, the subject matter of Example 4 can optionally include that the intermediary electrodes are spaced apart defining an interface.

In Example 10, the subject matter of Example 9 can optionally include that the catheter further includes a conductor disposed across the interface and in physical engagement with each intermediary electrode.

In Example 11, the subject matter of Example 10 can optionally include that the conductor is selected from one or more of the group consisting of at least one wire, and at least one welding bead.

In Example 12, the subject matter of Example 1 can optionally include that the first proximal ring electrode comprises a front surface comprising a plurality of spaced-apart extensions.

In Example 13, the subject matter of Example 12 can optionally include that the plurality of spaced-apart extensions are arranged circumferentially around at least part of the front surface of the first proximal ring electrode.

In Example 14, the subject matter of Example 13 can optionally include that the plurality of spaced-apart extensions of the first proximal electrode extend toward the first distal electrode and toward a spark gap defined between the first proximal and first distal ring electrodes comprising the first subsonic pressure wave generator.

In Example 15, the subject matter of Example 14 can optionally include that a front surface of the first distal ring electrode further comprises a plurality of spaced-apart extensions that extend toward the first proximal electrode and toward the spark gap.

In Example 16, the subject matter of Example 14 can optionally include that the plurality of spaced-apart extensions are arranged circumferentially around at least part of the front surface of the first distal ring electrode.

In Example 17, the subject matter of Example 2 can optionally include that at least the second proximal ring electrode comprises a front surface comprising a plurality of spaced-apart extensions.

In Example 18, the subject matter of Example 17 can optionally include that the plurality of spaced-apart extensions of the second proximal ring electrode extend toward the second distal electrode and toward a spark gap defined between the second proximal and second distal ring electrodes comprising the second subsonic pressure wave generator.

In Example 19, the subject matter of Example 18 can optionally include that the front surface of the first distal ring electrode further comprises a plurality of spaced-apart extensions that extend toward the first proximal electrode and toward the spark gap.

In Example 20, the subject matter of Example 19 can optionally include that the plurality of spaced-apart extensions are arranged circumferentially around at least part of the front surface of each of the second proximal electrode and the second distal electrode.

In Example 21, the subject matter of Example 1 can optionally include that at least one of the first proximal and distal ring electrodes defines a longitudinal channel, wherein the longitudinal channel is configured to receive a conductor that is in operative communication with the pulse generator.

In Example 22, the subject matter of Example 2 can optionally include that at least one of the second proximal and distal ring electrodes defines a longitudinal channel, wherein the longitudinal channel is configured to receive a conductor that is in operative communication with the pulse generator.

In Example 23, the subject matter of Example 1 can optionally include that the elongated catheter comprises a guide wire lumen therethrough.

In Example 24, the subject matter of Example 2 can optionally include that the catheter further includes a plurality of axially spaced-apart subsonic pressure wave generators.

In Example 25, the subject matter of Example 1 can optionally include that the voltage pulse provided by the pulse generator results in a bubble that expands in the fluid-filled balloon, with bubble expansion time measured in microseconds and resulting subsonic pressure wave.

In Example 26, the subject matter of Example 24 can optionally include that the axial spacing between the ring electrodes comprising each subsonic pressure wave generator in the plurality of axially-spaced apart subsonic pressure wave generators is 5mm or longer.

In Example 27, the subject matter of Example 24 can optionally include that the axial spacing distance between the ring electrodes comprising each subsonic pressure wave generator is related to the concentration of the conductive fluid in the balloon.

In Example 28, the subject matter of Example 1 can optionally include that the axial spacing distance between the first proximal and distal ring electrodes comprising the first subsonic pressure wave generator affects degradation of the first proximal and distal ring electrodes, wherein increasing axial spacing between the first proximal and distal ring electrodes reduces thermal degradation of the first proximal and distal ring electrodes by increasing a fraction of heat generated by the electrical arc that is configured to be dissipated into the conductive fluid.

In Example 29, the subject matter of Example 2 can optionally include that the axial spacing distance between the second proximal and distal ring electrodes comprising the second subsonic pressure wave generator affects degradation of the second proximal and distal ring electrodes, wherein increasing axial spacing between the second proximal and distal ring electrodes reduces thermal degradation of the second proximal and distal ring electrodes by increasing a fraction of heat generated by the electrical arc that is configured to be dissipated into the conductive fluid.

In Example 30, the subject matter of Example 24 can optionally include that the generation of subsonic pressure waves by at least adjacent ones of the plurality of subsonic pressure wave generators results in central pressure nodes located between the adjacently generated pressure waves.

In Example 31, the subject matter of Example 30 can optionally include that the catheter further includes a processor in communication with the pulse generator that is adapted to delay delivery of a first voltage pulse to one of the subsonic pressure wave generators in the plurality of subsonic pressure wave generators relative to delivery of a second voltage pulse to an adjacent subsonic pressure wave generator, wherein the adjacent subsonic pressure wave generators generate subsonic pressure waves and the delayed delivery of the voltage pulse generates a delayed subsonic pressure wave relative to the subsonic pressure wave generated by the second voltage pulse, and wherein the location of the central node between the adjacent subsonic pressure wave generators is dependent upon the magnitude of the delay.

In Example 32, the subject matter of Example 30 can optionally include that the catheter further includes a processor in communication with the pulse generator that is adapted to generate a voltage pulse of a first magnitude to one of the subsonic pressure wave generators in the plurality of subsonic pressure wave generators, and a voltage pulse of a second magnitude to an adjacent subsonic pressure wave generator in the plurality of subsonic pressure wave generators, wherein the first magnitude and the second magnitude are not equivalent, wherein the central pressure node between the adjacent subsonic pressure wave generators is not equidistant from each of the subsonic pressure wave generators, and wherein the location of the central node between adjacent subsonic pressure waves is dependent upon the difference between the first and second magnitudes.

In Example 33, the subject matter of Example 30 can optionally include that the catheter further includes a processor in communication with the pulse generator that is adapted to generate a voltage pulse of a first magnitude to one of the subsonic pressure wave generators in the plurality of subsonic pressure wave generators, and a voltage pulse of a second magnitude to an adjacent subsonic pressure wave generator in the plurality of subsonic pressure wave generators, wherein the first magnitude and the second magnitude are equivalent, wherein the location of the central pressure node between the adjacent subsonic pressure wave generators is substantially equidistant from each of the adjacent subsonic pressure wave generators.

In Example 34, the subject matter of Example 31 can optionally include that the processor comprises preprogrammed timing instructions for the initiation of voltage pulses and magnitude of the voltage pulses to the subsonic pressure wave generators.

In Example 35, the subject matter of Example 32 can optionally include that the voltage pulse initiation and/or magnitude of voltage pulses are configured to modify the location of individual subsonic pressure waves and location of central nodes between subsonic pressure waves generated by adjacent ones of the plurality of subsonic pressure wave generators.

In Example 36, the subject matter of Example 1 can optionally include that the ring electrodes comprise metal or semiconductor material.

In Example 37, the subject matter of Example 34 can optionally include that the ring electrodes are plated with a secondary alloy.

In Example 38, the subject matter of Example 35 can optionally include that a base metal comprises copper or beryllium copper.

In Example 39, the subject matter of Example 37 can optionally include that the plating comprises an electrochemically low-activity metal.

In Example 40, the subject matter of Example 37 can optionally include that the plating comprises one or more of the group consisting of: platinum, gold, tungsten, osmium, silver and nickel.

In Example 41, the subject matter of Example 36 can optionally include that the ring electrodes comprises one or more of the group consisting of: graphite, graphene, diamond, stainless steel and/or steel alloy(s).

Example 42 is a subsonic pressure wave generating angioplasty catheter. The subsonic pressure wave generating angioplasty catheter may include: an elongated carrier; an angioplasty balloon comprising a material and disposed near a distal end of the elongated catheter, wherein a distal end of the angioplasty balloon is sealed against the elongated catheter, the angioplasty balloon defining an interior region; a fluid channel in fluid communication with the interior region of the angioplasty balloon and a conductive fluid reservoir, adapted to inflate the balloon with the conductive fluid; at least one subsonic pressure wave generator disposed along the elongated carrier and within the interior region of the balloon, each of the at least one subsonic pressure wave generators comprising a proximal electrode and a distal electrode spaced axially from the proximal electrode, wherein at least one of the proximal and/or distal electrode comprises a plurality of points extending toward a spark gap defined between the proximal and distal electrodes; and a pulse generator in operative electrical communication with each of the at least one subsonic pressure wave generators, wherein application of a voltage pulse from the pulse generator to each of the at least one subsonic pressure wave generators results in an electrical arc through the conductive fluid and generation of subsonic pressure waves that pass through the conductive fluid and balloon material at subsonic speed.

Example 43 is a subsonic pressure wave generator. The subsonic pressure wave generator my include: an elongated carrier; a fluid-filled environment, wherein at least part of the elongated carrier is disposed; at least one subsonic pressure wave generator disposed along the elongated carrier and within the fluid-filled environment, each of the at least one subsonic pressure wave generators comprising a proximal electrode and a distal electrode spaced axially from the proximal electrode, wherein at least one of the proximal and/or distal electrode comprises a plurality of points extending toward a spark gap defined between the proximal and distal electrodes; and a pulse generator in operative electrical communication with each of the at least one subsonic pressure wave generators, wherein application of a voltage pulse from the pulse generator to each of the at least one subsonic pressure wave generators results in an electrical arc through the fluid and generation of subsonic pressure waves that pass through the fluid toward a target at subsonic speed.

Example 44 is a ring electrode configured for operative attachment to an elongated member. The ring electrode may include: a body portion defining a central aperture therethrough configured to receiving and engage the elongated member that extends longitudinally away from the body portion in a longitudinal proximal and a distal direction; a front surface and a rear surface, the front surface comprising a plurality of spaced-apart extensions extending away from the front surface in one of the longitudinal proximal or the distal directions, wherein each of the spaced-apart extensions are tapered; and a channel extending longitudinally from the front surface to the rear surface, configured to receive a conducting wire.

In Example 45, the subject matter of Example 44 can optionally include that the plurality of spaced-apart extensions are arranged circumferentially around at least part of the front surface of the first proximal ring electrode.

In Example 46, the subject matter of Example 44 can optionally include that each of the plurality of spaced-apart extensions terminates in a distal point.

In Example 47, the subject matter of Example 44 can optionally include that the channel comprises a carve-out of the body portion and wherein the body portion is configured to fully circumferentially engage the elongated member.

In Example 48, the subject matter of Example 44 can optionally include that channel defines a space between two spaced-apart ends of the body portion, wherein the body portion is configured to partially circumferentially engage the elongated member.

In Example 49, the subject matter of Example 44 can optionally include that the ring electrode comprises metal or semiconductor material.

In Example 50, the subject matter of Example 44 can optionally include that the ring electrode is plated with a secondary alloy.

In Example 51, the subject matter of Example 50 can optionally include that a base metal comprises copper or beryllium copper.

In Example 52, the subject matter of Example 50 can optionally include that the plating comprises an electrochemically low-activity metal.

In Example 53, the subject matter of Example 50 can optionally include that the plating comprises one or more of the group consisting of: platinum, gold, tungsten, osmium, silver and nickel.

In Example 54, the subject matter of Example 44 can optionally include that the ring electrodes comprises one or more of the group consisting of: graphite, graphene, diamond, stainless steel and/or steel alloy(s).

In Example 55, the subject matter of Example 44 can optionally include that the rear surface is substantially flat.

The description of the invention and its applications as set forth herein is illustrative and is not intended to limit the scope of the invention. Features of various embodiments may be combined with other embodiments within the contemplation of this invention. Variations and modifications of the embodiments disclosed herein are possible, and practical alternatives to and equivalents of the various elements of the embodiments would be understood to those of ordinary skill in the art upon study of this patent document. These and other variations and modifications of the embodiments disclosed herein may be made without departing from the scope and spirit of the invention.

## Claims

1. A balloon catheter comprising:
an elongated carrier;
a fluid-filled environment, wherein at least part of the elongated carrier is disposed;
a plurality of pairs of electrodes, each pair comprising a proximal electrode and a distal electrode, wherein the distal electrode in each pair of electrodes is spaced axially from the proximal electrode, wherein at least one of the proximal electrode and the distal electrode in each pair of the plurality of pairs of electrodes comprises a plurality of points extending toward a spark gap comprising a length that is defined between the proximal and distal electrodes; and
a pulse generator in operative electrical communication with each of the at least one of the proximal electrode and the distal electrode in each pair of electrodes of the plurality of pairs of electrodes,
wherein application of a voltage pulse from the pulse generator to any one of the proximal electrode and the distal electrode of a pair of electrodes in the plurality of pairs of electrodes results in an electrical arc through the fluid and generation of subsonic pressure waves that pass through the fluid toward a target at subsonic speed, and
wherein in the pulse generator is in operative communication with a processor comprising programmed instructions configured to initiate and control each applied voltage pulse.

2. The balloon catheter of claim 1, wherein the spark gap lengths of each of the plurality of pairs of electrodes are substantially equal.

3. The balloon catheter of claim 1, wherein the spark gap lengths of each of the plurality of pairs of electrodes are not equal.

4. The balloon catheter of claim 1, wherein the programmed instructions are configured to instruct a voltage magnitude that is the same for each voltage pulse.

5. The balloon catheter of claim 1, wherein the programmed instructions are configured to instruct voltage magnitudes that are not the same for each voltage pulse.

6. The balloon catheter of claim 1, wherein the programmed instructions of the voltage pulse applied to any one of the plurality of spark gap generators instruct simiultaneous initiation of voltage pulses to at least two of the plurality of subsonic wave generators, whereby the resulting electrical arcs occur at the same time.

7. The balloon catheter of claim 1, wherein the programmed instructions of the voltage pulse applied to any one of the plurality of spark gap generators instruct non-simultaneous initiation of voltage pulses to at least two of the pairs of electrodes in the plurality of electrode pairs, whereby the resulting electrics arcs do not occur at the same time.

8. The balloon catheter of claim 1, wherein the programmed instructions are configured to maintain an equal duration for each voltage pulse.

9. The balloon catheter of claim 1, wherein the programmed instructions are configured to maintain non-equal durations for at least two voltage pulses.

10. balloon catheter of claim 1, wherein at least two electrode pairs in the plurality of electrode pairs are connected in series.

11. The balloon catheter of claim 1, wherein at least two electrode pairs in the plurality of subsonic wave generators are connected in parallel.
